# EUROPÄISCHE PATENTANMELDUNG

(11) **EP 2 524 717 A1**
(43) Veröffentlichungstag der Anmeldung: **21.11.2012**
(21) Anmeldenummer: 11005147.1
(22) Anmeldetag: 24.06.2011
(51) Int. Cl.: A61N 1/36, A61N 1/04, A61H 39/00, A61H 39/06

(54) **Stimulationsvorrichtung**

(30) Priorität: 14.05.2011 DE 102011101521
(71) Anmelder: cerboMed GmbH, 91052 Erlangen (DE)
(72) Erfinder: Hartlep, Andreas, 83607 Holzkirchen (DE); Ellrich, Jens, 91094 Langensendelbach (DE)
(74) Vertreter: Gosdin, Michael

(57) **Zusammenfassung**

Die Erfindung betrifft eine Stimulationsvorrichtung (1) zur Stimulation mindestens eines Hautnervs im Kopf- und/oder Halsbereich eines Menschen, insbesondere dessen Stammes oder dessen Stämme, in seinem Verlauf von den rezeptiven Feldern an der Haut bis zu seiner Einmündung in den Hirnstamm, wobei die Stimulationsvorrichtung (1) mindestens ein Stimulationselement (2) umfasst, das mit am Kopf (K) des Menschen anbringbaren Haltemitteln (3) in einer Lage gehalten werden kann, so dass eine Stimulationsoberfläche des Stimulationselements auf dem Stamm des Nerven aufliegt oder an dem Stamm des Nerven anliegt. Hiermit wird es möglich, eine effiziente Nervenstimulation zu erreichen.

## Beschreibung

Die Erfindung betrifft eine Stimulationsvorrichtung zur Stimulation mindestens eines Hautnervs im Kopf- und/oder Halsbereich eines Menschen.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zu invasiven als auch zu non-invasiven Stimulation.

Bekannt ist in verschiedenen Variationen insbesondere die Stimulation beispielsweise des Vagusnervs mittels einer Stimulationsvorrichtung, die eine transkutane elektrische Stimulation vornimmt.

Die DE 10 2005 003 735 B4 offenbart eine Stimulationsvorrichtung, die einen bügelförmigen Fortsatz aufweist, an dessen Ende ein Elektrodenkopf samt Elektroden angeordnet ist. Der bügelförmige Fortsatz wird in den Gehörgang eingeführt, über die Wandung des Gehörkanals wird dann der Vagusnerv stimuliert.

In der DE 10 2006 023 824 B4 wird vorgesehen, die Stimulationsvorrichtung in der Pinna anzuordnen. Dabei können Elektroden eingesetzt werden, die am Ende elastischer Spannelemente positioniert sind, mit denen die Vorrichtung in der Pinna gehalten wird.

Es hat sich herausgestellt, dass die transkutane Stimulation der sensiblen Hautnerven an Gesicht, Kopf und Hals bei einer Vielzahl von neurologischen, psychiatrischen, internistischen und Hals-Nasen-Ohren-Erkrankungen nicht ausreicht. Demgemäß sind die vorbekannten Stimulationssysteme gegebenenfalls für die genannte Anwendung, aber auch für andere Anwendungen, nicht optimal.

Der Erfindung liegt daher die **Aufgabe** zugrunde, eine Stimulationsvorrichtung zur Stimulation der oben genannten Nerven des Kopf- bzw. Halsbereichs vorzusehen, mit der es möglich ist, bei Bedarf eine stärkere und effizientere Nervenstimulation zu erreichen.

Die **Lösung** dieser Aufgabe durch die Erfindung ist dadurch gekennzeichnet, dass die Stimulationsvorrichtung zur Stimulation mindestens eines Hautnervs im Kopf- und/oder Halsbereich eines Menschen, insbesondere von dessen Stamm oder dessen Stämmen, in seinem Verlauf von den rezeptiven Feldern an der Haut bis zu seiner Einmündung in den Hirnstamm ausgebildet ist, indem die Stimulationsvorrichtung mindestens ein Stimulationselement umfasst, das mit am Kopf des Menschen anbringbaren Haltemitteln in einer Lage gehalten werden kann, so dass eine Stimulationsoberfläche des Stimulationselements auf dem Stamm des Nerven aufliegt oder an dem Stamm des Nerven anliegt.

Die Stimulationsoberfläche des Stimulationselements liegt dabei bevorzugt flächig, insbesondere mit einer Auflagefläche zwischen 0,1 cm² und 20 cm², besonders bevorzugt mit einer Auflagefläche zwischen 0,2 cm² bis 10 cm², auf einem definierten Hautbereich auf.

Das Stimulationselement kann mindestens eine elektrische Elektrode zur Aufbringung eines transkutanen elektrischen Stimulationsreizes auf den definierten Hautbereich aufweisen. Das Stimulationselement kann aber auch mindestens eine die Haut des definierten Bereichs penetrierende (invasive) Elektrode zur Aufbringung eines elektrischen Stimulationsreizes aufweisen.

Eine alternative - gegebenenfalls aber auch additive - Ausgestaltung sieht vor, dass das Stimulationselement mindestens ein wärmeerzeugendes und/oder kälteerzeugendes Element zur Einleitung von Wärme und/oder Kälte in den definierten Hautbereich aufweist.

Eine weitere alternative - und wiederum gegebenenfalls auch additive - Konzeption sieht vor, dass das Stimulationselement mindestens ein Schwingungen erzeugendes Element zur Einleitung von Vibrationen in den definierten Hautbereich aufweist.

Gemäß einer möglichen Ausgestaltung umfassen die Haltemittel einen am Stimulationselement angeordneten oder angeformten bügelförmigen Abschnitt, der ausgebildet ist, um hinter der Pinna des Ohrs des Benutzers der Stimulationsvorrichtung eingehängt zu werden.

Die Haltemittel können weiterhin einen am Stimulationselement angeordneten oder angeformten Haltebügel umfassen, der ausgebildet ist, um einen Teil des Kopfs oder Halses, insbesondere um den Hinterkopf, umzulaufen und mindestens ein Stimulationselement, vorzugsweise zwei Stimulationselemente für jede Seite des Kopfs bzw. für jeden Nervenstamm, in Position zu halten.

Der Haltebügel kann dabei so ausgebildet sein, dass er bei bestimmungsgemäßem Gebrauch der Stimulationsvorrichtung das mindestens eine Stimulationselement, vorzugsweise zwei Stimulationselemente, mit Vorspannung an die Oberfläche des Kopfs drückt.

Die Haltemittel umfassen gemäß einer weiteren Ausführungsform der Erfindung einen bandförmigen Streifen, an dem mindestens ein Stimulationselement angeordnet ist, wobei der bandförmige Streifen insbesondere ausgebildet ist, über einen Teil des Kopfs flächig zu verlaufen. Der bandförmige Streifen kann dabei aus einem Material (z. B. aus Metall) bestehen, das plastisch verformbar ist. Damit kann der Streifen der individuellen Kopfform angepasst werden. In der plastisch umgeformten Lage kann das Material dann aber auch wieder elastische Eigenschaften aufweisen, um eine elastische Anlagekraft auf die Oberfläche des Kopfes an den gewünschten Behandlungsstellen zu erzeugen.

Der bandförmige Streifen kann dabei mindestens eine Gabelung aufweisen, so dass er mindestens drei Abschnitte aufweist, wobei insbesondere jeder Abschnitt bei bestimmungsgemäßem Gebrauch des Stimulationselements über einen Umfangsabschnitt des Kopfs verläuft. So kann die Stimulationsvorrichtung in einfacher Weise präzise positioniert werden.

Die Haltemittel können auch gemäß einer weiteren Ausführungsform der Erfindung einen schalenförmigen Körper umfassen, der eine konkave Oberfläche aufweist, um einen Abschnitt des Kopfes zu umfassen.

Die Haltemittel sind insbesondere so ausgebildet, dass mindestens ein Stimulationselement in mindestens einem der folgenden Bereiche des Kopfs gehalten bzw. positioniert werden kann:
- Bereich des Austrittspunkts des Nervus ophthalmicus (erster Ast des Nervus trigeminus) oberhalb des Randes der Augenhöhle im Bereich der Augenbrauen,
- Bereich des Austrittspunkts des Nervus maxilliaris (zweiter Ast des Nervus trigeminus) an der Wange unterhalb des Randes der Augenhöhle,
- Bereich des Austrittspunkts des Nervus mandibularis (dritter Ast des Nervus trigeminus) am Kinn seitlich der Kinnspitze,
- Bereich des Austrittspunkts des Nervus auricularis magnus am mittleren Hinterrand des Musculus sternocleidomastoideus,
- Bereich des Austrittspunkts des Nervus occipitalis minor am oberen Hinterrand des Musculus sternocleidomastoideus,
- Bereich des Austrittspunkts des Nervus occipitalis major am Ansatzpunkt der Halsstrecker am Hinterkopf,
- Bereich des Austrittspunkts der Hautäste des Nervus cervicalis III im Bereich dorsal und caudal des Verlaufs des Musculus sternocleidomastoideus,
- Bereich des Austrittspunkts der Nervus facialis im Bereich der unteren Ohrwurzel,
- Bereich des Austrittspunkts des Nervus vagus, insbesondere des aurikulären Vagusnervs, im Bereich hinter der Pinna.

Die Haltemittel können dabei insbesondere so ausgebildet sein, dass mindestens ein Stimulationselement jeweils auf dem linken und rechten Nervenaustrittspunkt gehalten werden kann.

Die vorgeschlagene Vorrichtung ist also primär für die Stimulation der Stämme der sensiblen Kopf- und Halsnerven konzipiert. Mit der vorgeschlagenen Lösung kann der jeweilige Nervenstamm mit einer ausreichenden Stimulationsenergie erreicht werden, obwohl er einige Millimeter unter der Haut liegt.

Der bevorzugte Stimulationsort ist jeweils der Stamm des Hautnervs am Nervenaustrittspunkt. Er soll jedoch im Verlauf des gesamten Weges stimuliert werden, von den sensiblen Hautästen in den rezeptiven Feldern bis zu seinem Eintritt in den Hirnstamm.

Soweit der Vagusnerv zu stimulieren ist, gilt: Der Stamm des Ramus Auricularis Nervi Vagi (RANV), auch Auricular Branch of the Vagus Nerve (ABVN) genannt, also der Ohrast des Vagusnervs, sammelt die sensiblen Nervenendigungen der diversen rezeptiven Felder, also beispielsweise aus der Cymba Conchae oder über dem Processus mastoideus, und führt sie durch den Canaliculus mastoideus und die Fissura petrotympanica ins Innere des Hirnschädels und zum Hirnstamm. Er versorgt sensibel die Haut der Innenseite der Pinna, des äußeren Gehörganges, einen Teil des Trommelfells sowie das oben angesprochene Hautareal über dem Processus mastoideus.

Es geht gemäß der vorliegenden Erfindung primär um die direkte Stimulation des Nervs und nicht um die Erregung kleiner Hautäste im rezeptiven Feld. Da der genaue anatomische Verlauf einer großen Variabilität unterliegt, ist darauf zu achten, gegebenenfalls den gesamten Bereich des Nervenaustrittspunktes mit dem Stimulationselement abzudecken. Es wird hierzu also eine möglichst große Fläche für die Stimulation vorgesehen, im Falle der Vagusnervstimulation insbesondere der gesamte Bereich zwischen Fissura und der Dorsalseite des äußeren Ohres.

Als Kontrolle der Stimulation dient das Empfinden des Patienten (Kribbeln in den jeweiligen, anatomisch zugeordneten rezeptiven Feldern).

Für die optimale Stimulation werden geeignete Stromformen und Stimulationssequenzen vorgesehen, mit denen die unter der Haut verlaufenden Nervenbündel auch mehrere Millimeter in die Tiefe hinein bis nahe an den Hirnstamm heran beeinflusst werden können.

Dabei haben sich Stimulationsfrequenzen im Bereich zwischen 1 und 100 Hz bewährt. Die Phasendauer von Stromimpulsen im Falle einer elektrischen Stimulation bewegt sich zumeist zwischen 10 und 1.000 µs.

Bei der elektrischen Nervenstimulation kann zum einen die invasive Stimulation vorgesehen werden.

Hier können kurze Nadeln vorgesehen werden (s. hierzu die US 2007/0150027 A1, auf die insoweit Bezug genommen wird), die zwar die

Hornschicht der Haut penetrieren, aber nicht bis hinab zur Lederhaut reichen, also keine Blutgefäße und Nerven erreichen. Da diese Homschicht normalerweise ein wirkungsvoller Isolator ist, gewinnt die Stimulation dadurch deutlich an Effizienz.

Möglich ist auch der Einsatz längerer Nadeln, die den Nervenstamm auf seinem Weg von den rezeptiven Feldern bis zum Hirnstamm erreichen und direkt stimulieren.

Des weiteren kommen spiralförmig um den Nervenstamm gelegte sog. Cuff Elektroden in Frage, die sich für eine effiziente Nervenstimulation eignen.

Alternativ oder additiv kann eine noninvasive, d. h. transkutane elektrische Nervenstimulation vorgesehen werden. Primär geeignet ist hierfür das Stimulationselement, das sich flächig auf den zu stimulierenden Bereich auflegt. Erwähnt wurde hierzu bereits die Positionierung mittels eines elastischen Haltebügels. Als Haltemittel wären aber beispielsweise auch Klebeelektroden möglich, die gegebenenfalls mit mindestens einer räumlich entfernten, größeren Gegenelektrode kombiniert werden.

Vorgesehen werden kann mindestens eine Stimulationselektrode, wobei eine Referenz-, Gegen- bzw. Neutralelektrode auch an einer entfernteren Stelle den Körper kontaktieren kann.

Weiterhin kann - insbesondere im Falle der Stimulation des Vagusnervs - eine Elektrode vorgesehen sein, die sowohl hinter dem Ohr (zur Stimulation des RANV-Nervenstamms) als auch über dem Mastoid stimuliert.

Möglich ist die Stimulation einseitig nur am linken oder rechten Nervenaustrittspunkt, aber auch an den linken und den rechten Nervenaustrittspunkten, jeweils an korrespondierenden Stellen.

Oben erwähnt wurde bereits ein beidohrig anzulegender Bügel (Haltebügel), der mit einem nach vorne gebogenen Haken (bügelförmiger Abschnitt) über bzw. hinter den Ohrmuscheln eingehängt und hinter dem Nacken vom einen zum anderen Ohr geführt wird.

Als zusätzliche Fixierung kann ein Teil des Haltebügels auch vorne um den Hals geführt sein.

Der jeweils über den Nervenstämmen verlaufende Teil der Stimulationsvorrichtung ist zur Bildung des Stimulationselements flächig verbreitert und trägt die Elektroden z. B. für den Bereich der zu beaufschlagenden Nervenaustrittspunkte.

Als Alternative kann der genannte Haltebügel auch nach Art eines Kopfhörers ausgeführt sein und bei bestimmungsgemäßem Gebrauch über den Scheitel des Benutzers verlaufen. In diesem Fall können sich die jeweiligen Enden des über den Scheitel verlaufenden Haltebügels über der Ohrwurzel in einen vorderen und einen hinteren Anteil gabeln. Der vordere Anteil dient dabei lediglich der Halterung, der hintere Anteil umfasst das Stimulationselement, im Falle der elektrischen Reizung des Nervs also die Elektroden.

Die Stimulationselemente können gemäß einer alternativen Lösung auch in einen hinter den Ohren weiter nach hinten und unten gerührten Brillenbügel integriert sein, wobei wiederum eine ein- oder beidseitige Stimulation möglich ist.

Eine weitere alternative Lösung stellt auf eine magnetische Halterung, insbesondere mittels eines Permanentmagneten, in bzw. an der Pinna ab. Hier kann eine anatomisch angeformte Magnetelektrode zum Einsatz kommen.

Eine hinter dem Ohr anzuordnende Elektrode bzw. das Stimulationselement kann dabei zumindest teilweise magnetisiert sein. In diesem Falle kann ein ferritisches Gegenstück in der Cymba conchae des Ohrs angeordnet werden.

Analog kann aber auch eine Elektrode bzw. ein Stimulationselement im Ohr zumindest teilweise magnetisiert sein, wobei dann ein ferritisches Gegenstück hinter dem Ohr angeordnet ist.

Die genannten Gegenstücke können jeweils selbst Elektroden tragen. Möglich ist es auch, dass - wie oben bereits angesprochen - neben dem Stimulationselement für die genannten Bereiche auch ein Stimulationselement mit zusätzlichen Elektroden an bzw. in der Pinna vorhanden ist, insbesondere in der Cymba conchae.

Aufgrund einer gegebenen großen Variabilität liegen bei manchen Patienten mehrere Stämme des jeweiligen Nervs vor, die sich bereits vor dem Austritt aus dem Hirnschädel aufgespalten haben. Daher wird vorliegend vom Stamm (wie es idealtypisch wäre) bzw. von den Stämmen des Nervs gesprochen.

In der Zeichnung sind Ausführungsbeispiele der Erfindung dargestellt. Es zeigen:
- Fig. 1: den Kopf eines Menschen, an dem die hier interessierenden Nervenbereiche eingezeichnet sind, die stimuliert werden können,
- Fig. 2: den Kopf gemäß Fig. 1, aus einer rückwärtigen Perspektive gesehen,
- Fig. 3: den Kopf in der Darstellung nach Fig. 1, wobei nun eine Stimulationsvorrichtung gemäß einer ersten Ausführungsform der Erfindung am Kopf angeordnet ist,
- Fig. 4: den Kopf gemäß Fig. 3, aus einer rückwärtigen Perspektive gesehen,
- Fig. 5: schematisch in perspektiver Ansicht den Kopf samt Ohr eines Menschen mit Markierung der für die Stimulation vorgesehenen Areale, um den Vagusnerv zu stimulieren,
- Fig. 6: in der Darstellung nach Fig. 5 eine Stimulationsvorrichtung, die nunmehr am Kopf angeordnet ist, und
- Fig. 7: die Stimulationsvorrichtung nach Fig. 6, wobei die Ansicht in Richtung "X" dargestellt ist.

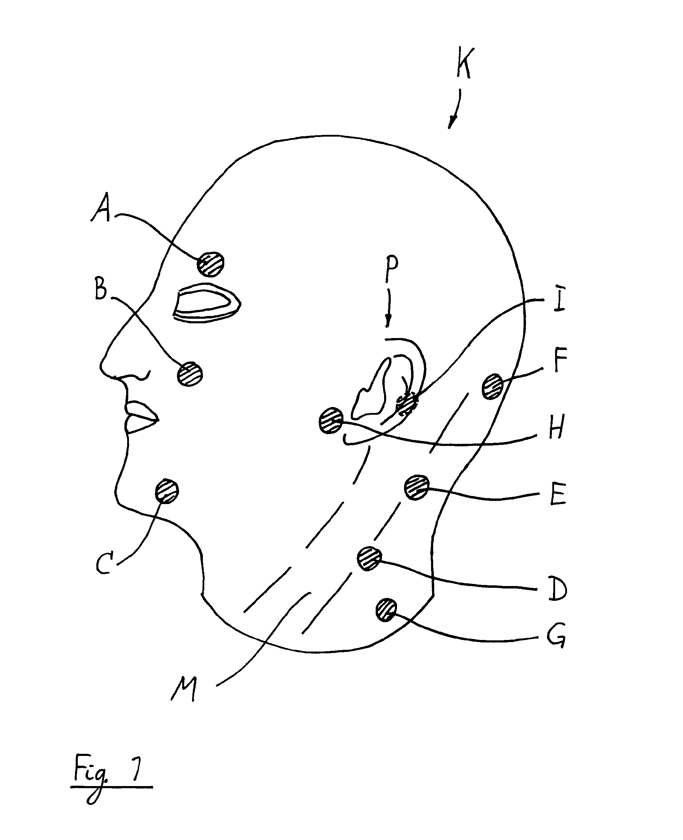

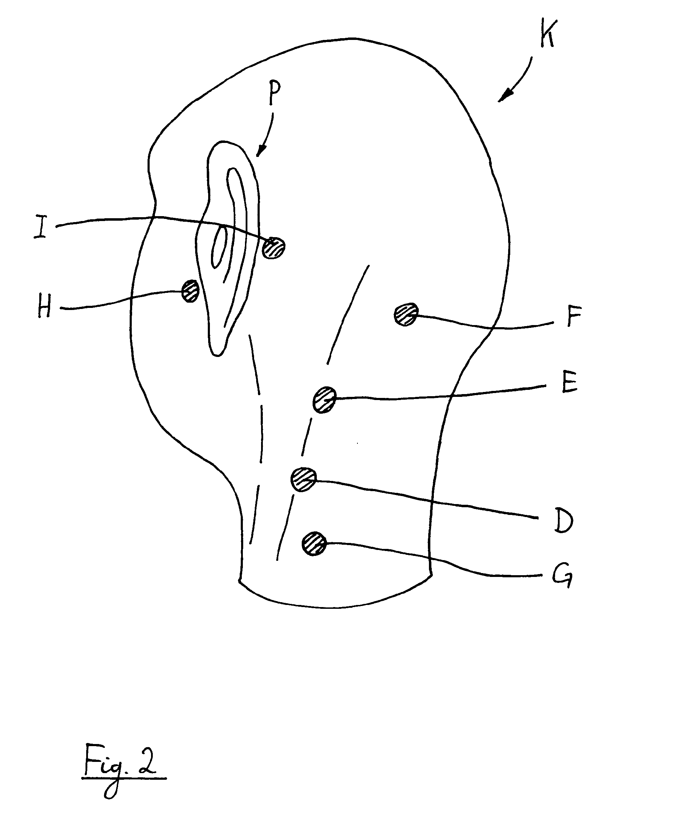

In Fig. 1 und Fig. 2 ist ein Kopf K eines Menschen zu sehen, an dem eine Stimulationsvorrichtung zur Stimulation mindestens eines Hautnervs im Kopf- und/oder Halsbereich angebracht werden soll, um insbesondere den Stamm oder die Stämme verschiedener Hautnerven im Verlauf des jeweiligen Nervs von den rezeptiven Feldern an der Haut bis zu seiner Einmündung in den Hirnstamm zu stimulieren.

In Frage kommen hierfür die folgenden in den Figuren markierten Nerven:
A Nervus ophthalmicus (erster Ast des Nervus trigeminus)
B Nervus maxillaris (zweiter Ast des Nervus trigeminus)
C Nervus mandibularis (dritter Ast des Nervus trigeminus)
D Nervus auricularis magnus
E Nervus occipitalis minor
F Nervus occipitalis major
G Nervus cervicalis III
H Nervus facialis
I Nervus vagus

Angedeutet ist in Fig. 1 noch der Musculus sternocleidomastoideus M.

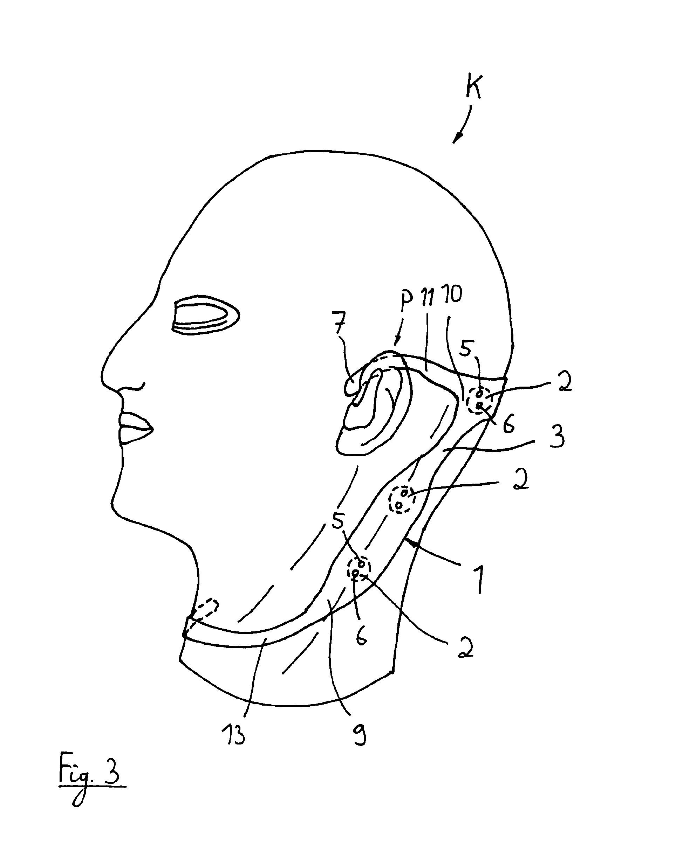

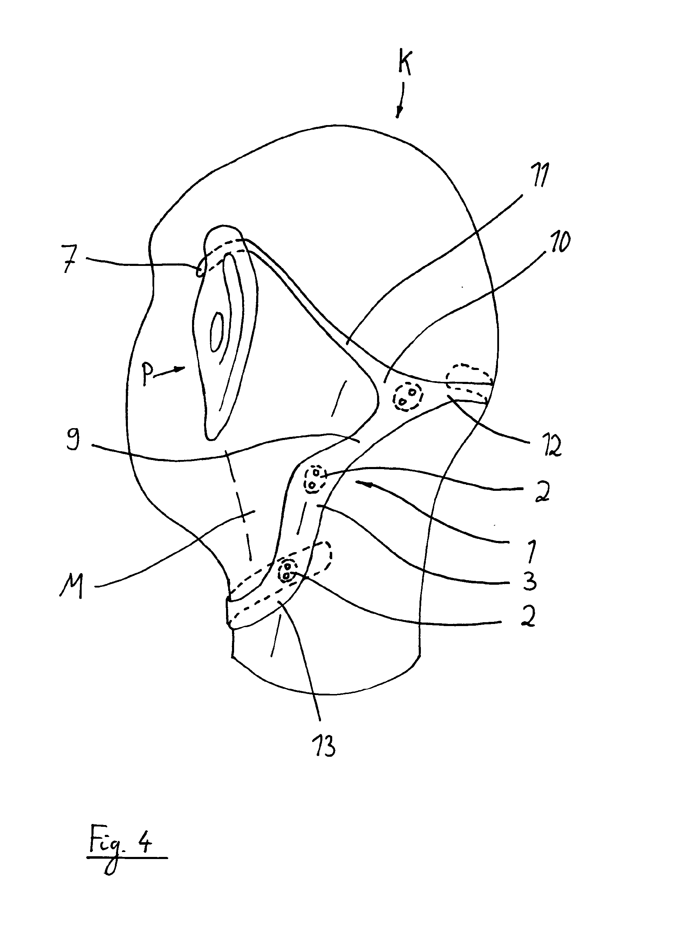

In den Figuren 3 und 4 ist zu sehen, wie am Kopf K eine Stimulationsvorrichtung 1 angeordnet ist, um vorliegend den Nervus auricularis magnus D, den Nervus occipitalis minor E und den Nervus occipitalis major F zu stimulieren.

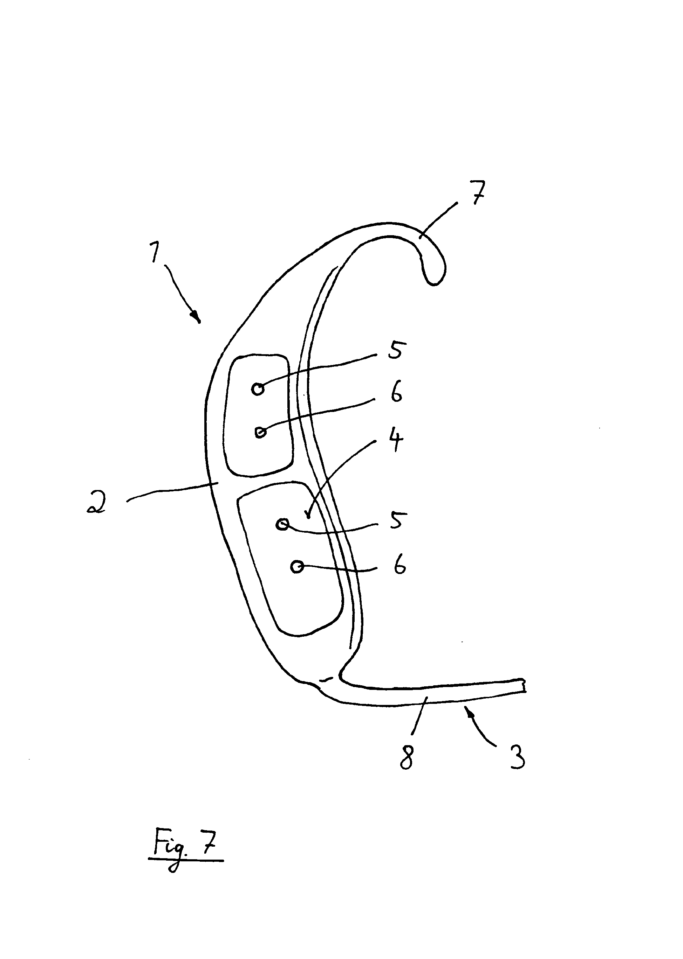

Die Stimulationsvorrichtung 1 weist hierzu flächig ausgebildete Stimulationselemente 2 auf, die eine solche flächige Ausdehnung aufweisen, dass die Austrittspunkte der genannten Nerven abgedeckt werden können. Die Stimulationsoberfläche 4 ist exemplarisch in Fig. 7 illustriert. Jedes Stimulationselement 2 weist jeweils mindestens eine Stimulationselektrode 5 und mindestens eine zugeordnete Referenzelektrode 6 auf, zwischen denen in bekannter Weise ein elektrischer Strom fließt, um eine transkutane Nervenstimulation durchführen zu können

Damit die Stimulationselemente 2 mit ihren Elektroden 5, 6 in der richtigen Position angeordnet und gehalten werden können, sind Haltemittel 3 vorgesehen, die im Ausführungsbeispiel gemäß Fig. 3 und 4 als bandförmiger Streifen 9 ausgeführt sind. Der bandförmige Streifen 9 weist vorliegend eine Gabelung 10 auf, der den Streifen 9 in drei Abschnitte 11, 12 und 13 gliedert. Der eine Abschnitt 11 des Streifens 9 hat endseitig einen bügelförmigen Endabschnitt 7, mit dem der Streifen 9 hinter die Pinna P eingehängt werden kann. Die anderen beiden Endbereiche der Abschnitt 12 bzw. 13 schlingen sich über einen Teil des Umfangs des Kopfs bzw. Halses herum und sorgen so für eine definierte Anlage der Stimulationsvorrichtung 1 am Kopf K.

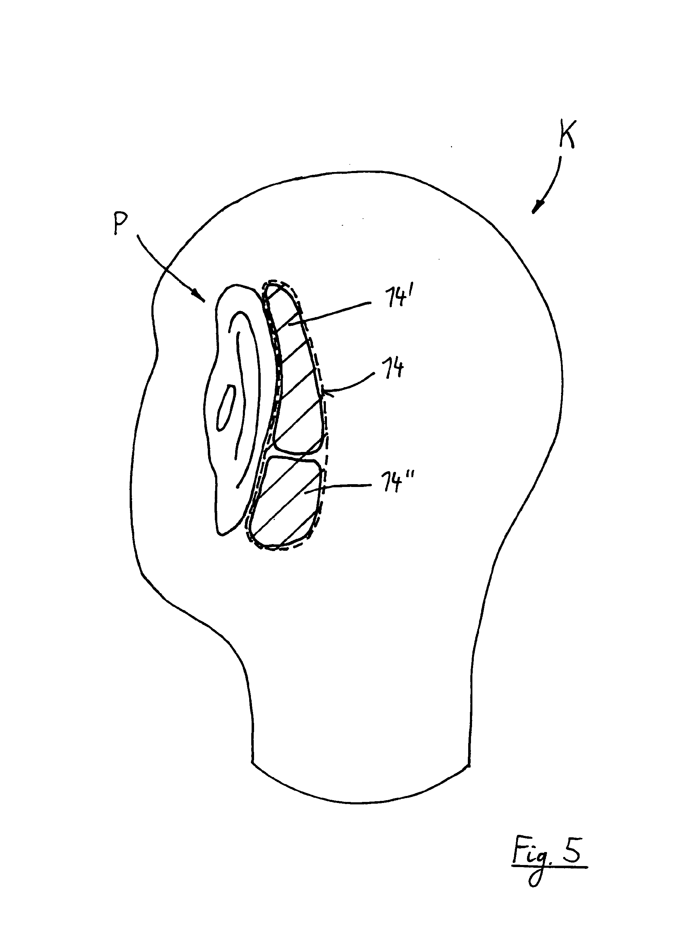

In Fig. 5 ist wiederum der Kopf K skizziert, wobei hier die Pinna P des Ohrs zu erkennen ist. Stimuliert werden soll hier gezielt der Vagusnerv I (s. Fig. 1 und 2). Dargestellt sind hier Bereiche hinter dem Ohr, die bestimmungsgemäß einer Stimulation unterzogen werden sollen, um den Vagusnerv an seinem Stamm bzw. an seinen Stämmen zu stimulieren.

Der insgesamt für die Stimulation in Frage kommende Bereich ist mit 14 markiert (s. gestrichelte Umrandung und den schraffierten Bereich). Der Bereich 14 umfasst allerdings zwei spezielle Bereiche, die spezifisch für die Stimulation vorgesehen werden können. Der eine Bereich 14' ist derjenige Abschnitt, wo der Stamm des Vagusnervs liegt. Der andere Bereich 14" liegt direkt über dem Felsenbein (Processus Mastoideus); demgemäß liegt hier das rezeptive Feld zur Stimulation des Vagusnervs. Vorliegend wird der Bereich 14' zur Stimulation vorgesehen.

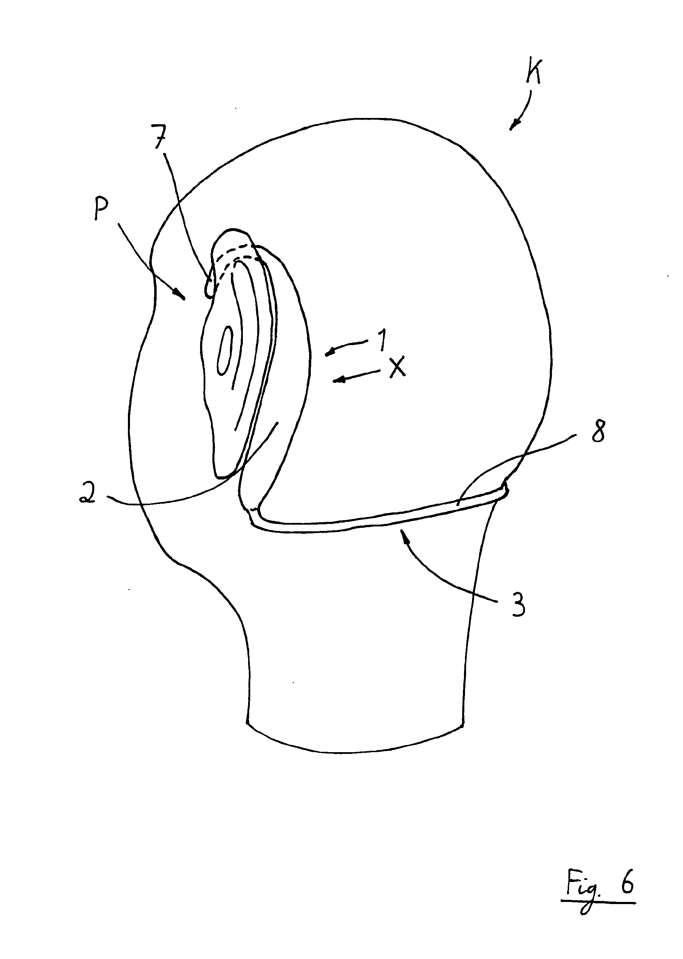

In Fig. 6 ist die Stimulationsvorrichtung 1 - jedenfalls ein Teil derselben - zu sehen, deren Kernstück wiederum ein Stimulationselement 2 ist. Das Stimulationselement 2 ist besonders gut in Fig. 7 zu erkennen, wo es - gesehen aus der Richtung X gemäß Fig. 6 - dargestellt ist. Das Stimulationselement 2 weist eine flächig ausgebildete Stimulationsoberfläche 4 auf, in der im Ausführungsbeispiel elektrische Elektroden 5 und 6 angeordnet sind. Die Darstellung der Elektroden 5, 6 in Fig. 7 ist nur sehr beispielhaft zu verstehen. Es kann eine Stimulation- und eine Referenzelektrode vorgesehen werden. Möglich ist es aber auch, dass mehrere Stimulations- und Referenzelektroden vorgesehen werden. Es ist auch möglich, dass eine oder mehrere Referenzelektroden an einem anderen Ort des Körpers platziert werden.

Das Stimulationselement 2 wird wiederum mit Haltemitteln 3 so in Position gehalten, dass bei bestimmungsgemäßem Gebrauch die Stimulationsoberfläche 4 des Stimulationselements 2 leicht an die Hautoberfläche des Bereichs 14, 14', 14" gedrückt wird.

Im Ausführungsbeispiel weisen die Haltemittel 3 hierfür einen Haltebügel 8 auf, der um den Hinterkopf bzw. Nacken des Benutzers umläuft und zwei Stimulationselemente für beide Ohren unter leichter elastischer Vorspannung in Position hält.

Des weiteren ist an dem Stimulationselement 2 wieder ein bügelförmiger Abschnitt 7 angeordnet, der vorgesehen ist, um hinter die Pinna P gehängt zu werden. Beide Elemente 7 und 8 der Haltemittel 3 wirken dabei synergetisch und sorgen für eine bequeme und angenehme Positionierung der Stimulationsvorrichtung 1 auf dem vorgesehenen Bereich 14.

In das dargestellte Stimulationselement 2 kann die gesamte Elektronik zur Steuerung der Nervenstimulation samt Energieversorgung integriert sein.

### Bezugszeichenliste:

- 1: Stimulationsvorrichtung
- 2: Stimulationselement
- 3: Haltemittel
- 4: Stimulationsoberfläche
- 5: Elektrode
- 6: Elektrode
- 7: bügelförmiger Abschnitt
- 8: Haltebügel
- 9: bandförmiger Streifen
- 10: Gabelung
- 11: Abschnitt
- 12: Abschnitt
- 13: Abschnitt
- 14: Bereich (Stimulationsbereich)
- 14': Bereich (Stimulationsbereich)
- 14": Bereich (Stimulationsbereich)
- K: Kopf
- P: Pinna
- A: Nervus ophthalmicus (erster Ast des Nervus trigeminus)
- B: Nervus maxillaris (zweiter Ast des Nervus trigeminus)
- C: Nervus mandibularis (dritter Ast des Nervus trigeminus)
- D: Nervus auricularis magnus
- E: Nervus occipitalis minor
- F: Nervus occipitalis major
- G: Nervus cervicalis III
- H: Nervus facialis
- I: Nervus vagus
- M: Musculus sternocleidomastoideus

## Patentansprüche

1. Stimulationsvorrichtung (1) zur Stimulation mindestens eines Hautnervs im Kopf- und/oder Halsbereich eines Menschen, insbesondere dessen Stammes oder dessen Stämme, in seinem Verlauf von den rezeptiven Feldern an der Haut bis zu seiner Einmündung in den Hirnstamm, wobei die Stimulationsvorrichtung (1) mindestens ein Stimulationselement (2) umfasst, das mit am Kopf (K) des Menschen anbringbaren Haltemitteln (3) in einer Lage gehalten werden kann, so dass eine Stimulationsoberfläche des Stimulationselements auf dem Stamm des Nerven aufliegt oder an dem Stamm des Nerven anliegt.

2. Stimulationsvorrichtung nach Anspruch 1, **dadurch gekennzeichnet, dass** die Stimulationsoberfläche (4) des Stimulationselements (2) flächig, insbesondere mit einer Auflagefläche von mindestens 2 cm², auf einem definierten Hautbereich aufliegt.

3. Stimulationsvorrichtung nach Anspruch 1 oder 2, **dadurch gekennzeichnet, dass** das Stimulationselement (2) mindestens eine elektrische Elektrode (5, 6) zur Aufbringung eines transkutanen elektrischen Stimulationsreizes auf den definierten Hautbereich aufweist.

4. Stimulationsvorrichtung nach Anspruch 1 oder 2, **dadurch gekennzeichnet, dass** das Stimulationselement (2) mindestens eine die Haut des definierten Bereichs penetrierende Elektrode zur Aufbringung eines elektrischen Stimulationsreizes aufweist.

5. Stimulationsvorrichtung nach Anspruch 1 oder 2, **dadurch gekennzeichnet, dass** das Stimulationselement (2) mindestens ein wärmeerzeugendes und/oder kälteerzeugendes Element zur Einleitung von Wärme und/oder Kälte in den definierten Hautbereich aufweist.

6. Stimulationsvorrichtung nach Anspruch 1 oder 2, **dadurch gekennzeichnet, dass** das Stimulationselement (2) mindestens ein Schwingungen erzeugendes Element zur Einleitung von Vibrationen in den definierten Hautbereich aufweist.

7. Stimulationsvorrichtung nach einem der Ansprüche 1 bis 6, **dadurch gekennzeichnet, dass** die Haltemittel (3) einen am Stimulationselement (2) angeordneten oder angeformten bügelförmigen Abschnitt (7) umfassen, der ausgebildet ist, um hinter der Pinna (P) des Ohrs eingehängt zu werden.

8. Stimulationsvorrichtung nach einem der Ansprüche 1 bis 7, **dadurch gekennzeichnet, dass** die Haltemittel (3) einen am Stimulationselement (2) angeordneten oder angeformten Haltebügel (8) umfassen, der ausgebildet ist, um einen Teil des Kopfs (K), insbesondere um den Hinterkopf, umzulaufen und mindestens ein Stimulationselement (2), vorzugsweise zwei Stimulationselemente (2) für jedes Seite des Kopfs (K), in Position zu halten.

9. Stimulationsvorrichtung nach Anspruch 8, **dadurch gekennzeichnet, dass** der Haltebügel (8) ausgebildet ist, um bei bestimmungsgemäßem Gebrauch der Stimulationsvorrichtung (1) das mindestens eine Stimulationselement (2), vorzugsweise zwei Stimulationselemente (2), mit Vorspannung an die Oberfläche des Kopfs (K) zu drücken.

10. Stimulationsvorrichtung nach einem der Ansprüche 1 bis 7, **dadurch gekennzeichnet, dass** die Haltemittel (3) einen bandförmigen Streifen (9) umfassen, an dem mindestens ein Stimulationselement (2) angeordnet ist, wobei der bandförmige Streifen (9) insbesondere ausgebildet ist, über einen Teil des Kopfs (K) flächig zu verlaufen.

11. Stimulationselement nach Anspruch 10, **dadurch gekennzeichnet, dass** der bandförmige Streifen (9) aus einem Material besteht, das plastisch verformbar ist.

12. Stimulationselement nach Anspruch 10 oder 11, **dadurch gekennzeichnet, dass** der bandförmige Streifen (9) mindestens eine Gabelung (10) aufweist, so dass er mindestens drei Abschnitte (11, 12, 13) aufweist, wobei insbesondere jeder Abschnitt (11, 12, 13) bei bestimmungsgemäßem Gebrauch des Stimulationselements über einen Umfangsabschnitt des Kopfs (K) verläuft.

13. Stimulationsvorrichtung nach einem der Ansprüche 1 bis 7, **dadurch gekennzeichnet, dass** die Haltemittel (3) einen schalenförmigen Körper umfassen, der eine konkave Oberfläche aufweist, um einen Abschnitt des Kopfes (K) zu umfassen.

14. Stimulationselement nach einem der Ansprüche 1 bis 13, **dadurch gekennzeichnet, dass** die Haltemittel (3) ausgebildet sind, um mindestens ein Stimulationselement (2) in mindestens einem der folgenden Bereiche des Kopfs (K) zu halten:
- Bereich des Austrittspunkts des Nervus ophthalmicus (A) (erster Ast des Nervus trigeminus) oberhalb des Randes der Augenhöhle im Bereich der Augenbrauen,
- Bereich des Austrittspunkts des Nervus maxilliaris (B) (zweiter Ast des Nervus trigeminus) an der Wange unterhalb des Randes der Augenhöhle,
- Bereich des Austrittspunkts des Nervus mandibularis (C) (dritter Ast des Nervus trigeminus) am Kinn seitlich der Kinnspitze,
- Bereich des Austrittspunkts des Nervus auricularis magnus (D) am mittleren Hinterrand des Musculus sternocleidomastoideus (M),
- Bereich des Austrittspunkts des Nervus occipitalis minor (E) am oberen Hinterrand des Musculus sternocleidomastoideus (M),
- Bereich des Austrittspunkts des Nervus occipitalis major (F) am Ansatzpunkt der Halsstrecker am Hinterkopf,
- Bereich des Austrittspunkts der Hautäste des Nervus cervicalis III (G) im Bereich dorsal und caudal des Verlaufs des Musculus sternocleidomastoideus (M),
- Bereich des Austrittspunkts der Nervus facialis (H) im Bereich der unteren Ohrwurzel,
- Bereich des Austrittspunkts des Nervus vagus (I), insbesondere des aurikulären Vagusnervs, im Bereich hinter der Pinna (P).

15. Stimulationselement nach Anspruch 14, **dadurch gekennzeichnet, dass** die Haltemittel (3) ausgebildet sind, mindestens ein Stimulationselement (2) jeweils auf dem linken und rechten Nervenaustrittspunkt zu halten.
